# EUROPÄISCHE PATENTSCHRIFT

(11) **EP 2 910 934 B1**
(45) Veröffentlichungstag und Bekanntmachung des Hinweises auf die Patenterteilung: **07.07.2021**
(21) Anmeldenummer: 15151641.6
(22) Anmeldetag: 19.01.2015
(51) Int. Cl.: G01N 21/84, G01B 11/06, G01N 21/896

(54) **Vorrichtung zur Inspektion eines mit einer beschichteten Oberfläche versehenen Materials und entsprechendes Verfahren**
Device for inspecting a material provided with a coated surface and corresponding method
Dispositif d'inspection d'un matériau doté d'une surface revêtue et procédé correspondant

(30) Priorität: 20.01.2014 DE 102014100594
(43) Veröffentlichungstag der Anmeldung: 26.08.2015
(73) Patentinhaber: Isra Surface Vision GmbH, 45699 Herten (DE)
(72) Erfinder: Kubiak, Rolf, 44269 Dortmund (DE)
(74) Vertreter: Keil & Schaafhausen Patentanwälte PartGmbB

(56) Entgegenhaltungen:
- EP-A2- 2 390 656
- DE-A1- 4 331 355
- DE-A1- 19 509 345
- US-A1- 2007 258 093
- US-B1- 7 369 240
- None

## Beschreibung

Die vorliegende Erfindung betrifft eine Vorrichtung zur Inspektion eines mit einer beschichteten Oberfläche versehenen Materials, vorzugsweise Glas, sowie ein entsprechendes Verfahren.

Oberflächenbeschichtungen auf Materialien, insbesondere Glas, sollen die Eigenschaften dieser Materialien verbessern oder verändern, beispielsweise als Anti-Reflex-Beschichtungen oder Wärmeschutzbeschichtungen. Derartige Schichten sind beispielsweise dünne Metallschichten oder dielektrische Schichten. Beschichtete Oberflächen, insbesondere solche mit mehreren Schichten, bilden Interferenzen aus (Interferenzschichten), welche dazu führen, dass die Oberflächen unter verschiedenen Betrachtungswinkeln verschiedene Farben zeigen. Diese Farben sind insbesondere durch die Dicke der Interferenzschichten vorgegeben.

Bei großen Glasflächen, z.B. bei Glasfassaden, können derartige Farbeffekte Designfunktion übernehmen. Hierbei möchte man (neben gegebenenfalls anderen Funktionen dieser Schichten) erreichen, dass die gesamte Glasfassade homogen in einer einzigen, vorgegebenen Farbe erscheint (oft grün oder blau), wobei Farbabweichungen hiervon als unschön empfunden werden. Dies bedeutet, dass das von der Materialoberfläche reflektierte Licht eine bestimmte Farbe, d.h. ein bestimmtes Spektrum an Wellenlängen aufweist. Bei derartigen großen Flächen ist ferner gewünscht, dass sich die Fassadenfarbe auch bei Änderung des Betrachtungswinkels, zum Beispiel beim Vorbeigehen an dieser Fassade, nicht verändert.

Bei der Herstellung derartiger Materialien mit beschichteten Oberflächen können Fehler auftreten, die dazu führen, dass die Farbe der Oberfläche nicht mehr homogen aussieht. Hierbei werden zwei Fehlertypen unterschieden, nämlich den Fehlertyp 1, bei dem sich die Farbe bei Betrachtung unter konstantem Betrachtungswinkel ändert. Der Fehlertyp 2 ergibt sich, wenn die Oberfläche zwar unter einem konstanten Betrachtungswinkel die gleiche Farbe aufweist, sich die Farbe der Oberfläche jedoch mit Änderung des Betrachtungswinkels ändert. Dieser Effekt tritt bei Mehrfachbeschichtungen prinzipiell immer auf, es wird jedoch angestrebt, diesen Fehlertyp durch Einhaltung bestimmter Schichtdicken so klein wie möglich zu halten. Die Unterscheidung in die oben genannten zwei Fehlertypen wird vorgenommen, weil beide Fehlertypen verschiedene Ursachen aufweisen und verschiedene Gegenmaßnahmen erfordern. Daher ist es auch wichtig, bei einer Farbabweichung zu wissen, welcher Fehlertyp vorliegt.

Die Druckschrift DE 195 09 345 A1 beschreibt ein Verfahren zum Erkennen und Bewerten von örtlich begrenzten Fehlern in einer selektiv reflektierenden Oberflächenschicht auf einem transparenten Substrat durch die digitale Datenverarbeitung eines von der Videokamera gelieferten Bildes. Bei dem Verfahren wird eine Beleuchtungseinrichtung eingesetzt, mit der eine Schicht einer Glasscheibe mittels weißem Licht unter einem schrägen Einfallswinkel beleuchtet wird. Die Schicht ist eine Wärmestrahlen reflektierende Schicht auf der Oberfläche der Glasscheibe. Das von der Beleuchtungseinrichtung kommende weiße Licht wird durch die Schicht jeweils unter einem dem Einfallswinkel entsprechenden Ausfallswinkel reflektiert. Die so reflektierten Lichtstrahlen werden auf einem ebenen Projektionsschirm mit weißer Oberfläche sichtbar gemacht. Weiter sind vier Farbvideokameras vorgesehen, welche derart senkrecht auf den Projektionsschirm ausgerichtet sind, dass jede Videokamera ein Viertel der Gesamthöhe des auf dem Projektionsschirm entstehenden Bildes erfasst, wobei die vier erfassten Felder aneinander anschließen oder überlappen. Die vier Farbvideokameras sind identisch aufgebaut und speichern jeweils in ihren den drei Grundfarben entsprechenden Matrix-Bildspeichern für jeden Bildpunkt den digitalen Leuchtdichtewert der jeweiligen Grundfarbe des entsprechenden Bildpunktes im Sichtfeld der jeweiligen Kamera. Die in den Bildspeichern jeder Kamera enthaltenen Leuchtdichtesignale werden anschließend jeweils einer Filterstufe zugeleitet, welche eine zweidimensionale Filterung der eingehenden Signale vornimmt, um die Signale zu entstören und zu glätten und repräsentative Mittelwerte für die Leuchtdichte einer Farbe in einem ausgewählten Feld zu erhalten. Die von den Filterstufen gelieferten Signale werden danach einer Farbvergleichsstufe zugeleitet, in der ein Farbvergleich jeder Grundfarbe an jeder Stelle des Bildes mit den einem farbneutralen oder weißen Bild entsprechenden Signalen durchgeführt wird. Anhand dieses Vergleichs wird beim Überschreiten eingegebener Grenzwerte ein Signal erzeugt, sodass fehlerhafte Schichten erkannt und gegebenenfalls aussortiert werden können.

Bisher wurde die Überprüfung der Oberflächen mit mehreren Spektrometern durchgeführt, welche die Farbe des an der Oberfläche reflektierten Lichts unter mindestens zwei Betrachtungswinkeln ermittelt haben. Da in jedem Spektrometer die Farbe lediglich eines kleinen Bereichs der Oberfläche bestimmt werden kann, muss die Oberfläche mit diesen Spektrometern punktweise abgescannt werden. Dies ist jedoch sehr aufwendig und langwierig, so dass nach einer einfacheren und kostengünstigeren Lösung für die Farbinspektion von beschichteten Oberflächen gesucht wird.

Neben den oben beschriebenen Farbfehlern können bei der Produktion solcher Oberflächen noch eine Vielzahl anderer Fehler auftreten, die hinsichtlich der Farbe keine Auswirkungen haben, wie zum Beispiel optische Fehler, Blasen, Einschlüsse, Kratzer. Hochwertige Oberflächen, wie beispielsweise Bauglas für Glasfassaden, werden heute fast immer optisch auf diese anderen Fehler inspiziert. Diese Inspektionsanlagen arbeiten mit einer hohen Auflösung, da die gesuchten Fehler klein sind. Die Farbinformation aus dem reflektierten Licht wird für die Untersuchung derartiger Fehler nicht benötigt, so dass die für die Erkennung derartiger Fehler verwendeten optischen Aufnahmeeinrichtungen die Farbinformation nicht erfassen.

Die Aufgabenstellung der vorliegenden Erfindung besteht somit darin, eine Vorrichtung anzugeben, mit der die Farbinspektion eines mit einer beschichteten Oberfläche versehenen Materials einfach und kostengünstig durchführbar ist. Entsprechend besteht die Aufgabe darin, ein einfaches und kostengünstiges Verfahren zur Farbinspektion beschichteter Oberflächen anzugeben.

Die obige Aufgabenstellung wird durch die Vorrichtung mit den Merkmalen des Anspruchs 1 sowie das in Anspruch 9 angegebene Verfahren gelöst.

Insbesondere weist die erfindungsgemäße Vorrichtung eine über der Oberfläche des beschichteten Materials angeordnete Lichtquelle, welche Licht in einem vorgegebenen Wellenlängenbereich in Richtung der Oberfläche abgibt,
und mindestens eine erste Kamera auf, wobei die erste Kamera zur Ermittlung eines Farbwerts des erfassten Lichts eingerichtet ist,
wobei die erste Kamera oberhalb einer auf der Oberfläche liegenden Linie derart geneigt zur Lichtquelle angeordnet dass sie die spiegelnde Reflexion des von der Lichtquelle ausgesandten Lichts an der beschichteten Oberfläche im Bereich der Inspektionslinie sieht, und einen ersten (großen) Öffnungswinkel besitzt derart,
dass von einem ersten Punkt der Linie reflektiertes Licht der Lichtquelle unter einem ersten Betrachtungswinkel und von einem zu dem ersten Punkt beabstandeten zweiten Punkt der Linie unter einem zweiten Betrachtungswinkel separat erfassbar ist, wobei der erste Betrachtungswinkel und der zweite Betrachtungswinkel verschieden sind, wobei die mindestens eine erste Kamera einen ersten Farbwert des von dem ersten Punkt reflektierten Lichts und einen zweiten Farbwert des von dem zweiten Punkt reflektierten Lichts ermittelt,
wobei eine mit der ersten Kamera verbundene Auswerteeinrichtung vorgesehen ist, welche den ersten Farbwert und den zweiten Farbwert oder deren Differenz jeweils mit einem bestimmten, vorgegebenen Farbsollwert oder mit einem bestimmten, vorgegebenen Farbsollwert-Bereich vergleicht und welche den Farbwert des reflektierten Lichts von verschiedenen Punkten der Inspektionslinie zusätzlich abhängig von deren zugehörigen Betrachtungswinkel auswertet.

Zusätzlich können auch die Farbwerte für der erste und der zweite Betrachtungswinkel ermittelt werden, die gleich sind.

Die von der Kamera zu einem Zeitpunkt abgetastete Linie reflektierten Lichts der Lichtquelle, mit der mindestens der erste und der zweite Punkt, d.h. viele Punkte, der Oberfläche gleichzeitig erfasst werden, wird im Folgenden auch als Inspektionslinie oder Scanlinie bezeichnet.

Die erfindungsgemäße Vorrichtung ermöglicht bei Verwendung lediglich einer Kamera auf der Basis des Vergleichs des ersten Farbwerts und des zweiten Farbwerts von zwei verschiedenen Punkten der Inspektionslinie miteinander, welche durch die Kamera unter verschiedenen Betrachtungswinkeln beobachtet werden, die Feststellung, ob Farbabweichungen vorliegen. Wenn Farbabweichungen festgestellt werden, liegt mit hoher Wahrscheinlichkeit Fehlertyp 2 vor.

In einem bevorzugten Ausführungsbeispiel ist die Auswerteeinrichtung derart eingerichtet, dass die Differenz des ersten Farbwerts des ersten Punktes der Inspektionslinie und des zweiten Farbwerts des zweiten Punktes der Inspektionslinie mit dem bestimmten, vorgegebenen Farbsollwert oder dem bestimmten, vorgegebenen Farbsollwert-Bereich verglichen werden, wobei der erste Punkt und der zweite Punkt durch die Kamera unter dem gleichen oder annähernd dem gleichen Betrachtungswinkel beobachtet werden (d.h. es handelt sich um voneinander beabstandete Punkte, wobei der erste Betrachtungswinkel und der zweite Betrachtungswinkel gleich oder annähernd gleich sind und wobei die Betrachtungswinkel in eine verschiedene Richtung entlang der Inspektionslinie gemessen werden). Dieses Verfahren kann mit einem oder mehreren Punktpaaren durchgeführt werden, die jeweils unter dem gleichen oder annähernd gleichen Betrachtungswinkel beobachtet werden. Bei diesem Ausführungsbeispiel kann festgestellt werden, dass mit hoher Wahrscheinlichkeit Fehlertyp 1 vorliegt, der Fehlertyp 2 kann nicht ausgeschlossen werden.

Um auch das Vorliegen des Fehlertyps 1 zuverlässig detektieren zu können, ist bei der erfindungsgemäßen Vorrichtung zusätzlich mindestens eine zweite Kamera vorgesehen, wobei die zweite Kamera ebenfalls zur Ermittlung eines Farbwerts des erfassten Lichts eingerichtet ist, wobei die zweite Kamera oberhalb der auf der Oberfläche liegenden Inspektionslinie und geneigt zur Lichtquelle angeordnet ist, wobei die zweite Kamera einen zweiten (großen) Öffnungswinkel besitzt derart, dass von jedem Punkt der Inspektionslinie reflektiertes Licht der Lichtquelle vorzugsweise gleichzeitig durch die erste Kamera und durch die zweite Kamera erfassbar ist, wobei die erste Kamera und die zweite Kamera jeweils den Farbwert des von jedem Punkt der Inspektionslinie reflektierten Lichts ermitteln, wobei die mit der ersten Kamera und der zweiten Kamera verbundene Auswerteeinrichtung die so ermittelten Farbwerte der ersten Kamera und der zweiten Kamera zu jedem Punkt der Inspektionslinie zu einem Vergleich mit dem bestimmten, vorgegebenen Farbsollwert oder dem bestimmten, vorgegebenen Farbsollwert-Bereich heranzieht.

Die erfindungsgemäße Vorrichtung beinhaltet somit eine erste Kamera und mindestens eine zweite Kamera, welche von jedem Punkt der Inspektionslinie einen Farbwert der ersten Kamera und einen Farbwert der zweiten Kamera des von der Oberfläche reflektierten Lichts der Lichtquelle erfassen. Hierbei besitzen beide Kameras einen so großen Öffnungswinkel, dass das reflektierte Licht von jedem Punkt der Inspektionslinie von der ersten Kamera und der zweiten Kamera erfasst werden kann. Die erste Kamera und die zweite Kamera sind derart vorzugsweise nebeneinander angeordnet, dass das von jedem Punkt der Inspektionslinie reflektierte Licht der Lichtquelle durch jede Kamera unter einem anderen Betrachtungswinkel aufgenommen wird. Hierbei kann abhängig von der Anordnung der Kameras die Situation entstehen, in der sich der Betrachtungswinkel der ersten Kamera nicht oder nur sehr geringfügig von dem Betrachtungswinkel der zweiten Kamera unterscheidet. In diesem Fall ist es vorteilhaft, für einen solchen Bereich der Inspektionslinie eine dritte Kamera vorzusehen, welche die Punkte des Bereichs der Inspektionslinie unter einem dritten Betrachtungswinkel erfasst. Diese Vorgehensweise wird weiter unten im Detail beschrieben.

Als Betrachtungswinkel gilt im Rahmen der vorliegenden Erfindung der Raumwinkel der Blickrichtung der jeweiligen Kamera auf den entsprechenden Punkt der Inspektionslinie zu der in diesem Punkt angeordneten Senkrechten auf der Oberfläche, alternativ kann auch der Winkel der Blickrichtung der jeweiligen Kamera auf den entsprechenden Punkt der Inspektionslinie, projiziert auf die Ebene, die durch die in diesem Punkt angeordnete Senkrechte auf der Oberfläche und die Inspektionslinie aufgespannt wird, herangezogen werden.

Weiter wird im Rahmen der vorliegenden Erfindung der Ausdruck "geneigt (schräg) zur Lichtquelle" so verstanden, dass die Kamera derart angeordnet ist, dass sie die spiegelnde Reflexion des von der Lichtquelle ausgesandten Lichts an der beschichteten Oberfläche im Bereich der Inspektionslinie sieht.

Mit der Auswerteeinrichtung, an welche die von der ersten Kamera und der mindestens einen zweiten Kamera ermittelten Farbwerte von jedem Punkt der Inspektionslinie übermittelt werden, werden nun jeweils die Farbwerte des von jedem Punkt der Inspektionslinie reflektierten, von der ersten Kamera und von der zweiten Kamera erfassten Lichts jeweils mit einem bestimmten, vorgegebenen Farbsollwert oder einem bestimmten, vorgegebenen Farbsollwert-Bereich verglichen, um eine gegebenenfalls vorliegende Farbabweichung zu erkennen. Mit diesem Verfahren kann insbesondere festgestellt werden, ob eine Farbabweichung gemäß Fehlertyp 2 vorliegt. Werden zusätzlich die Farbwerte der Punkte analysiert, welche den gleichen Betrachtungswinkel zu der ersten und der zweiten Kamera (entweder in Bezug auf die gleiche Kamera oder in Bezug auf die verschiedenen Kameras) aufweisen, kann zudem das Vorliegen des Fehlertyps 1 geprüft werden. Hierbei wertet die Auswerteeinrichtung gegebenenfalls den Farbwert des reflektierten Lichts von verschiedenen Punkten der Inspektionslinie zusätzlich abhängig von deren zugehörigen Betrachtungswinkel aus.

Um für alle Punkte der Inspektionslinie Farbwerte unter einem ersten Betrachtungswinkel und einem zweiten Betrachtungswinkel zu erhalten, deren Betrachtungswinkel eine möglichst große Differenz aufweisen, ist es insbesondere von Vorteil, wenn mindestens drei Kameras vorgesehen sind, welche oberhalb der Inspektionslinie vorzugsweise nebeneinander angeordnet sind. Hierbei sind die Öffnungswinkel der Kameras derart überlappend angeordnet, dass das von jedem Punkt der Inspektionslinie reflektierte Licht durch mindestens zwei der drei Kameras, dabei von den zwei Kameras jeweils unter einem anderen Betrachtungswinkel, erfasst wird.

Es ist weiterhin von Vorteil, wenn die erste Kamera und die mindestens eine zweite Kamera auf einem gemeinsamen Träger angeordnet sind. Hierdurch ist die erfindungsgemäße Vorrichtung besonders kleinbauend, was vorteilhaft ist, da der Platz in den Produktionslinien sehr begrenzt und jede zusätzliche Inspektionsstelle teuer ist.

Weiterhin von Vorteil ist, wenn die erste Kamera und/oder die mindestens eine zweite Kamera jeweils als eine Zeilenkamera oder eine Flächenkamera ausgebildet ist, wobei die Zeilenkamera eine Vielzahl von in einer Reihe angeordneten Lichterfassungselementen und die Flächenkamera eine Vielzahl von in einer Fläche angeordneten Lichterfassungselementen (z.B. Fotodioden) besitzt. Die Kamerazeile einer Zeilenkamera beziehungsweise eine Zeile einer Flächenkamera kann das reflektierte Licht mindestens eines Bereichs einer Inspektionslinie gleichzeitig erfassen. Mittels einer Flächenkamera kann der Farbwert des reflektierten Lichts mehrerer Inspektionslinien gleichzeitig aufgenommen werden. Die Zeilenkamera und die Flächenkamera sind dabei Farbkameras, welche den Farbwert jedes erfassten Punkts bestimmen. Hierbei nimmt jedes Lichterfassungselement (z.B. jede Fotodiode) der Kamera für einen bestimmten Punkt der Inspektionslinie einen Farbwert auf, wobei die Ausdehnung des "Punktes" entlang der Inspektionslinie durch die gerätespezifische Auflösung der Kamera bestimmt wird. Jedem Punkt der Inspektionslinie, der Licht reflektiert, das durch ein einziges Lichterfassungselement aufgenommen wird, ist ein bestimmter Betrachtungswinkel zugeordnet, der sich aus der Blickrichtung der Kamera auf diesen Punkt ergibt. Daher werden allen Punkten der Inspektionslinie, die durch alle Lichterfassungselemente einer Zeilenkamera gleichzeitig aufgenommen werden, jeweils andere Betrachtungswinkel zugeordnet. Gleiches gilt analog für eine Flächenkamera.

Um insbesondere hinsichtlich Fehlertyp 2 eine zuverlässige Aussage über die Qualität des Materials beziehungsweise der Oberflächenbeschichtung zu erhalten, ist es von Vorteil, wenn der Unterschied des ersten Betrachtungswinkels und des zweiten Betrachtungswinkels möglichst groß ist, insbesondere wenn der Betrachtungswinkelunterschied, auch bei Verwendung von mindestens zwei Kameras für die Betrachtung eines Punktes, mindestens 10°, vorzugsweise mindestens 15°, besonders bevorzugt mindestens 20° beträgt.

Eine effiziente und genaue Farbinspektion von großen beschichteten Oberflächen ist möglich, wenn das Material relativ zu der ersten und der zweiten Kamera und zu der Lichtquelle in Richtung einer Vorschubrichtung quer zur Inspektionslinie bewegbar ist, wobei vorzugsweise die Lichtquelle und/oder die erste Kamera und/oder die zweite Kamera in oder gegen Vorschubrichtung geneigt sind, um die spiegelnde Reflexion der Lichtquelle zu erfassen. Vorzugsweise beträgt der Neigungswinkel der Kamera zu der Senkrechten auf der beschichteten Oberfläche mindestens 20°.

Erfindungsgemäß ist die Lichtquelle vorzugsweise als im Wesentlichen linienförmige Lichtquelle, welche sich vorzugsweise parallel zur Inspektionslinie erstreckt und vorzugsweise weißes Licht abgibt, ausgebildet. Besonders bevorzugt wird eine Lichtquelle bestehend aus einer Reihe oder mehreren, in Vorschubrichtung hintereinander angeordneten Reihen von LEDs eingesetzt.

Wenn die Inspektionslinie vorzugsweise die gesamte Breite des Materials senkrecht zur Vorschubrichtung überdeckt, ist ein einfaches und schnelles Scannen der gesamten Oberfläche möglich.

Wie oben bereits erläutert wurde, ist es von Vorteil, wenn in der erfindungsgemäßen Vorrichtung mindestens eine dritte Kamera vorgesehen ist, welche zur Ermittlung eines dritten Farbwerts des erfassten Lichts eingerichtet ist, wobei die dritte Kamera oberhalb der Inspektionslinie und geneigt zur Lichtquelle angeordnet ist, wobei die dritte Kamera einen dritten Öffnungswinkel besitzt derart, dass zumindest für die Punkte der Inspektionslinie in einem bestimmten Bereich der Inspektionslinie zusätzlich das reflektierte Licht der Lichtquelle (18) erfassbar und der zugehörige Farbwert ermittelbar ist, wobei die Auswerteeinrichtung für jeden Punkt des bestimmten Bereichs der Inspektionslinie den Betrachtungswinkel der dritten Kamera mit dem Betrachtungswinkel der ersten Kamera und/oder dem Betrachtungswinkel der zweiten Kamera vergleicht und für den entsprechenden Punkt des bestimmten Bereichs diejenigen zwei Farbwerte der ersten Kamera, der zweiten Kamera und der dritten Kamera zum Vergleich mit dem bestimmten, vorgegebenen Farbsollwert oder dem bestimmten, vorgegebenen Farbsollwert-Bereich heranzieht, für welche die Differenz der zugehörigen Betrachtungswinkel am größten ist.

Bei diesem Ausführungsbeispiel wird bei dem Vergleich der Betrachtungswinkel in jedem Punkt der Inspektionslinie der jeweils minimale und der maximale Betrachtungswinkel aller Kameras ermittelt, die in dem jeweiligen Punkt Farbwerte bestimmt haben. Von der Auswerteeinrichtung werden dann die Farbwerte der beiden Kameras für den Vergleich mit dem Farbsollwert beziehungsweise dem Farbsollwert-Bereich herangezogen, deren zugehörige Kamera den minimalen Betrachtungswinkel und deren Kamera den maximalen Betrachtungswinkel aufweist.

In einem weiteren bevorzugten Ausführungsbeispiel wird durch die Auswerteeinrichtung in jedem Punkt der Inspektionslinie jeweils eine Farbwertdifferenz von zwei Farbwerten zweier bestimmter, vorgegebener Kameras (d.h. der ersten, zweiten oder dritten Kamera, z.B. der Kameras mit dem größten Betrachtungswinkelunterschied), die das reflektierte Licht dieses Punkts beobachten, ermittelt und diese Farbwertdifferenz mit einem bestimmten, vorgegebenen Farbwertdifferenz-Sollwert oder einem bestimmten, vorgegebenen Farbwertdifferenz-Sollwertbereich verglichen, gegebenenfalls abhängig von der Differenz der zugehörigen Betrachtungswinkel. Dieses Auswerteverfahren ist insbesondere für die Feststellung eines Farbfehlers Typ 2 von Vorteil und schneller als eine Einzelauswertung der Farbwerte der zwei Kameras für jeden Punkt der Inspektionslinie. Dieses Ausführungsbeispiel für eine erfindungsgemäße Vorrichtung ist insbesondere dann anwendbar, wenn bekannt ist, dass die zu untersuchende beschichtete Oberfläche die größten Farbdifferenzen unter gewissen Winkeln ausbildet, so dass nur bestimmte, kleine Winkelbereiche genauer betrachtet werden müssen.

Das erfindungsgemäße Verfahren zur Inspektion eines mit einer beschichteten Oberfläche versehenen Materials, vorzugsweise Glas, wobei eine über der Oberfläche angeordneten Lichtquelle, welche Licht in einem vorgegebenen Wellenlängenbereich in Richtung der Oberfläche abgibt, und mindestens eine erste Kamera vorgesehen sind, wobei die erste Kamera zur Ermittlung eines Farbwerts des erfassten Lichts eingerichtet ist, wobei die erste Kamera oberhalb einer auf der Oberfläche liegenden Inspektionslinie geneigt zur Lichtquelle angeordnet ist, dass sie die spiegelnde Reflexion des von der Lichtquelle ausgesandten Lichts an der beschichteten Oberfläche im Bereich der Inspektionslinie sieht, und einen ersten (großen) Öffnungswinkel besitzt, weist insbesondere die folgenden Schritte auf:
- mittels der ersten Kamera wird das von einem ersten Punkt der Inspektionslinie reflektierte Licht der Lichtquelle unter einem ersten Betrachtungswinkel und das von einem zu dem ersten Punkt beabstandeten zweiten Punkt der Inspektionslinie unter einem zweiten Betrachtungswinkel separat erfasst, wobei der erste Betrachtungswinkel und der zweite Betrachtungswinkel verschieden sind, wobei die erste Kamera einen ersten Farbwert des von dem ersten Punkt reflektierten Lichts und einen zweiten Farbwert des von dem zweiten Punkt reflektierten Lichts ermittelt,
- wobei mittels einer mit der ersten Kamera verbundenen Auswerteeinrichtung der erste Farbwert und der zweite Farbwert oder deren Differenz jeweils mit einem bestimmten, vorgegebenen Farbsollwert oder einem bestimmten, vorgegebenen Farbsollwert-Bereich verglichen werden, mittels der Auswerteeinrichtung der Farbwert des reflektierten Lichts von verschiedenen Punkten der Inspektionslinie zusätzlich abhängig von deren zugehörigen Betrachtungswinkel ausgewertet wird.

Das erfindungsgemäße Verfahren ist einfach durchführbar und weist zudem die Vorteile der oben erläuterten erfindungsgemäßen Vorrichtung auf. Das angegebene Verfahren ist, wie oben bereits ausgeführt wurde, insbesondere dafür geeignet, mit hoher Wahrscheinlichkeit bei einer Farbabweichung Farbfehler des Fehlertyps 1 festzustellen, wenn Punkte der Inspektionslinie der Farbinspektion zugrunde gelegt werden, welche von der Kamera unter dem gleichen Betrachtungswinkel beobachtet werden. Bei dieser Konstellation kann der Fehlertyp 2 nicht ausgeschlossen werden. Werden verschiedenen Punkte der Inspektionslinie mit verschiedenen Betrachtungswinkeln zur Farbinspektion herangezogen und Farbabweichungen festgestellt, kann das Vorliegen des Fehlertyps 2 lediglich mit hoher Wahrscheinlichkeit ermittelt werden.

Analog zu obigen Ausführungen zu der erfindungsgemäßen Vorrichtung ist es von erfindungsgemäß vorgesehen, dass mindestens eine zweite Kamera vorhanden ist, wobei die zweite Kamera ebenfalls zur Ermittlung eines Farbwerts des erfassten Lichts eingerichtet ist, wobei die zweite Kamera oberhalb der auf der Oberfläche liegenden Inspektionslinie und geneigt zur Lichtquelle angeordnet ist, wobei die zweite Kamera einen zweiten (großen) Öffnungswinkel besitzt, wobei durch die erste Kamera und durch die zweite Kamera von jedem Punkt der Inspektionslinie reflektiertes Licht der Lichtquelle vorzugsweise gleichzeitig erfasst und jeweils der Farbwert des von jedem Punkt der Inspektionslinie reflektierten Lichts ermittelt wird sowie durch die Auswerteeinrichtung mit dem bestimmten, vorgegebenen Farbsollwert oder dem bestimmten, vorgegebenen Farbsollwert-Bereich verglichen wird.

Hinsichtlich des Verfahrens ist zudem von Vorteil, wenn mittels der Auswerteeinrichtung der Farbwert des reflektierten Lichts von verschiedenen Punkten der Inspektionslinie zusätzlich abhängig von deren zugehörigen Betrachtungswinkel ausgewertet werden, um den Fehlertyp 1 zu ermitteln. Hierbei wird demnach der Betrachtungswinkel mit in die Analyse einbezogen und untersucht, ob bei einem bestimmten Betrachtungswinkel der gewünschte Farbwert erzielt wird.

In einem bevorzugten Ausführungsbeispiel des erfindungsgemäßen Verfahrens ist mindestens eine dritte Kamera vorgesehen, welche zur Ermittlung eines Farbwerts des erfassten Lichts eingerichtet ist, wobei die dritte Kamera oberhalb der Inspektionslinie und geneigt zur Lichtquelle angeordnet ist, wobei die dritte Kamera einen dritten (großen) Öffnungswinkel besitzt, wobei durch die dritte Kamera zumindest für die Punkte der Inspektionslinie in einem bestimmten Bereich der Inspektionslinie zusätzlich das reflektierte Licht der Lichtquelle erfasst und der zugehörige Farbwert ermittelt wird, wobei durch die Auswerteeinrichtung für jeden Punkt des bestimmten Bereichs der Inspektionslinie der Betrachtungswinkel der dritten Kamera mit dem Betrachtungswinkel der ersten Kamera und/oder dem Betrachtungswinkel der zweiten Kamera verglichen wird und für den entsprechenden Punkt des bestimmten Bereichs diejenigen zwei Farbwerte der ersten Kamera, der zweiten Kamera und der dritten Kamera zum Vergleich mit dem bestimmten, vorgegebenen Farbsollwert oder dem bestimmten, vorgegebenen Farbsollwert-Bereich herangezogen werden, für welche die Differenz der zugehörigen Betrachtungswinkel am größten ist. Wie oben bereits erläutert wurde, ist es durch diese Ausgestaltung möglich, eine große Betrachtungswinkel-Differenz zu erzielen, da beispielsweise für jeden Punkt der Inspektionslinie jeweils der Farbwert der Kamera mit dem minimalen Betrachtungswinkel und der Farbwert der Kamera mit dem maximalen Betrachtungswinkel für den Vergleich mit dem vorgegebenen Farbsollwert oder Farbsollwert-Bereich herangezogen werden kann.

Wie oben bereits erläutert wurde, ist es ferner von Vorteil, wenn mittels der Auswerteeinrichtung in jedem Punkt der Inspektionslinie jeweils eine Farbwertdifferenz von zwei Farbwerten zweier bestimmter Kameras, die das reflektierte Licht dieses Punkts beobachten, ermittelt und diese Farbwertdifferenz mit einem bestimmten, vorgegebenen Farbwertdifferenz-Sollwert oder einem bestimmten, vorgegebenen Farbwertdifferenz-Sollwertbereich verglichen wird, gegebenenfalls zusätzlich abhängig von dem jeweiligen Betrachtungswinkel.

Der Vergleich des ersten Farbwertes und/oder des zweiten Farbwertes und/oder gegebenenfalls des dritten Farbwertes mit dem Farbsollwert oder dem Farbsollwert-Bereich wird in der Auswerteeinheit vorzugsweise basierend auf dem Lab-Farbraum durchgeführt, welcher durch die Norm EN ISO 11664-4 festgelegt ist. Dieser Farbraum (Farbmodell) deckt den Bereich der wahrnehmbaren Farben ab und ist auf der Grundlage der Gegenfarbentheorie konstruiert. Hierbei wird der Lab-Farbraum durch ein dreidimensionales Koordinatensystem beschrieben, wobei die a-Achse den Grün- oder Rotanteil einer Farbe, die b-Achse den Blau- oder Gelbanteil einer Farbe und die L-Achse die Helligkeit (Luminanz) der Farbe mit Werten zwischen 0 und 100 beschreibt. Dieser Farbraum wurde früher auch als CIELAB-Farbenraum bezeichnet. Für den Vergleich der Farbwerte beziehungsweise der Farbdifferenzwerte mit dem Farbsollwert oder dem Farbsollwertbereich beziehungsweise dem Farbwertdifferenz-Sollwert oder dem Farbwertdifferenz-Sollwertbereich müssen gegebenenfalls die Farbwerte, die der Auswerteeinrichtung von der ersten und/oder der zweiten Kamera und/oder der dritten Kamera übermittelt werden, in den Lab-Farbraum umgerechnet werden.

Die Erfindung wird nachfolgend anhand von Ausführungsbeispielen und/oder Bezugnahme auf die Figuren näher erläutert. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbezügen.

Es zeigen schematisch:
- Figur 1: ein erstes Ausführungsbeispiel einer erfindungsgemäßen Vorrichtung in einer Ansicht von vorn,
- Figur 2: das Ausführungsbeispiel gemäß Figur 1 in einer Ansicht von der Seite,
- Figur 3: ein zweites Ausführungsbeispiel einer erfindungsgemäßen Vorrichtung in einer Ansicht von vorn,
- Figur 4: ein Diagramm, das den Betrachtungswinkelverlauf jeder Kamera des ersten Ausführungsbeispiels in Abhängigkeit von dem Ort des Punktes auf der Inspektionslinie zeigt,
- Figur 5: ein Diagramm, das aus dem in Fig. 4 gezeigten Betrachtungswinkelverlauf für alle Kameras den Verlauf kleiner Betrachtungswinkel und den Verlauf großer Betrachtungswinkel sowie die Differenz aus diesen für die Kameras gemäß dem ersten Ausführungsbeispiel in Abhängigkeit von dem Ort des Punktes auf der Inspektionslinie zeigt,
- Figur 6: ein Diagramm, das den Betrachtungswinkelverlauf jeder Kamera des zweiten Ausführungsbeispiels in Abhängigkeit von dem Ort des Punktes auf der Inspektionslinie zeigt,
- Figur 7: ein Diagramm, das aus dem in Fig. 6 gezeigten Betrachtungswinkelverlauf für alle Kameras den Verlauf kleiner Betrachtungswinkel und den Verlauf großer Betrachtungswinkel sowie die Differenz aus diesen für die Kameras gemäß dem zweiten Ausführungsbeispiel in Abhängigkeit von dem Ort des Punktes auf der Inspektionslinie zeigt,
- Fig. 8: ein drittes Ausführungsbeispiel einer erfindungsgemäßen Vorrichtung während der Inspektion eines gleichmäßig beschichteten Materials in einer Ansicht von vorn,
- Fig. 9: das Ausführungsbeispiel gemäß Fig. 8 während der Inspektion eines ungleichmäßig aber symmetrisch zur optischen Achse der Kamera beschichteten Materials in einer Ansicht von vorn und
- Fig. 10: das Ausführungsbeispiel gemäß Fig. 8 während der Inspektion eines keilförmig beschichteten Materials in einer Ansicht von vorn.

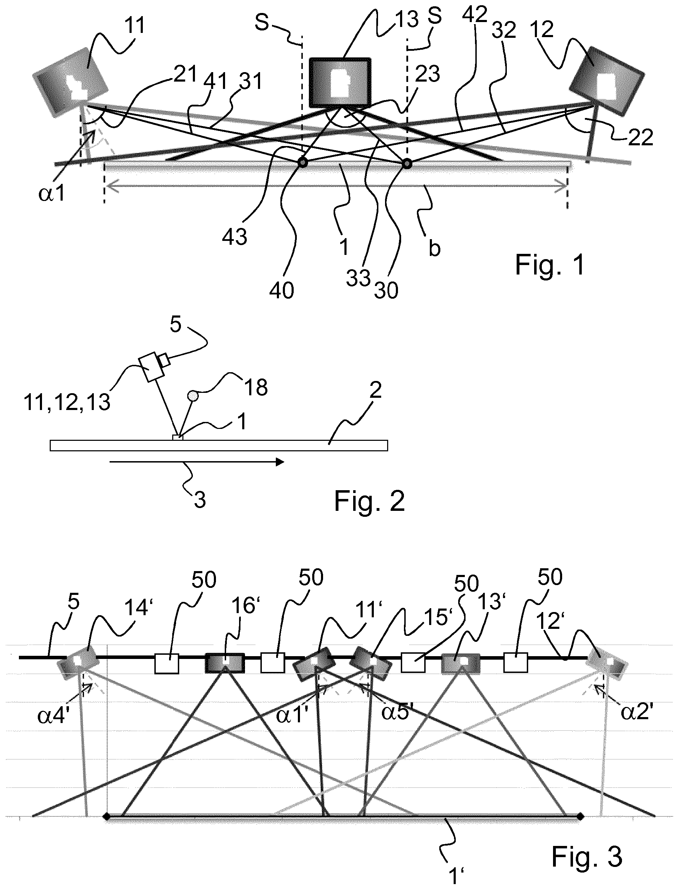

Das in Fig. 1 dargestellte Ausführungsbeispiel einer erfindungsgemäßen Vorrichtung zeigt die Inspektionslinie 1 auf der Oberfläche des in Fig. 2 gezeigten Materials in Form eines mit einer Oberflächenbeschichtung (z.B. einer Anti-Reflex-Beschichtung oder einer Wärmeschutzbeschichtung) versehenen Glases 2, welche durch eine erste Kamera 11, eine zweite Kamera 12 und eine dritte Kamera 13 beobachtet wird. Dabei wird das Material 2 relativ zu den feststehenden Kameras 11, 12 ,13 und der über der Oberfläche des Materials 2 angeordneten Lichtquelle 18, welche beispielsweise durch eine parallel zur Inspektionslinie angeordnete Reihe von LEDs ausgebildet ist, die weißes Licht aussenden, in Richtung der mit dem Pfeil 3 veranschaulichten Bewegungsrichtung (siehe Fig. 2) bewegt. Die Inspektionslinie 1 mit der Breite b verläuft quer zur Bewegungsrichtung und erstreckt sich dabei über die gesamte Breite des Glases 2, so dass die gesamte Oberfläche des Glases 2 hinsichtlich der Farbe des reflektierten Lichts inspiziert werden kann. Die Kameras 11, 12 und 13 sind jeweils als Farbkameras und als Zeilenkameras ausgebildet.

Wie in Fig. 1 zu erkennen ist, weist die erste Kamera 11 einen ersten Öffnungswinkel 21, die zweite Kamera 12 einen zweiten Öffnungswinkel 22 und die dritte Kamera 13 einen dritten Öffnungswinkel 23 auf. Die erste Kamera 11, die zweite Kamera 12 und die dritte Kamera 13 sind nebeneinander auf einem stangenförmigen Träger 5 (siehe Fig. 2) angeordnet. Die erste Kamera 11 und die zweite Kamera 12 sind in einem bestimmten, vorgegebenen Winkel (hinsichtlich Kamera 11 ist der Winkel α1 in Fig. 1 eingezeichnet) zur Senkrechten auf der Oberfläche des Materials 2 in der Ebene, die durch die Senkrechte und die Inspektionslinie 1 aufgespannt wird, verkippt. Hierdurch kann jede dieser beiden Kameras 11, 12 die Reflexion des von der Lichtquelle 18 ausgesandten und in jedem Punkt der Inspektionslinie 1 reflektierten Lichts unter einem anderen Betrachtungswinkel erfassen. Fig. 2 zeigt, dass die Kameras 11, 12, 13 in Bewegungsrichtung 3 verkippt sind, so dass die Reflexion des von der Lichtquelle 18 ausgesandten Lichts an der Inspektionslinie 1 auch durch die Kameras 11, 12, 13 beobachtbar ist. An dieser Stelle soll darauf hingewiesen werden, dass die Inspektionslinie 1 anders als in Fig. 2 schematisch dargestellt, in der Oberfläche des Glases 2 liegt, wobei die Inspektionslinie 1 vorzugsweise eine gewisse Ausdehnung in Richtung quer zur Inspektionslinie 1 auf der Oberfläche aufweist, um Höhenunterschiede, die das Glas 2 an seiner Oberfläche aufweisen kann, auszugleichen und um eine Farbinspektion trotz derartiger Höhenunterschiede zu ermöglichen. Hierfür ist es notwendig, dass das an der Oberfläche im Bereich der Inspektionslinie reflektierte Licht der Lichtquelle 18 die jeweilige Kamera 11, 12, 13 erreicht.

Jede Kamera 11, 12 und 13 erfasst das von der Lichtquelle 18, die oberhalb der Inspektionslinie 1 angeordnet ist, erzeugte und an der Oberfläche des Glases 2 reflektierte Licht. Da der erste Öffnungswinkel 21 der ersten Kamera 11 und der zweite Öffnungswinkel 22 der zweiten Kamera 12 so groß gewählt ist, dass diese Kameras 11, 12 jeden Punkt der Inspektionslinie 1 sehen, kann der Farbwert jedes Punkts der Inspektionslinie 1 durch die Kameras 11, 12 unter zwei verschiedenen Betrachtungswinkeln erfasst werden.

Im mittleren Bereich der Inspektionslinie 1 ist zudem die dritte Kamera 13 angeordnet, welche von den Punkten der Inspektionslinie 1, die innerhalb des Öffnungswinkels 23 der dritten Kamera 13 liegen, zusätzlich den Farbwert des von der Oberfläche des Glases 2 reflektierten Lichts unter einem dritten Betrachtungswinkel erfasst.

Beispielsweise sieht die erste Kamera 11 einen ersten Punkt 30 der Inspektionslinie 1 entlang eines ersten Sehstrahls 31, die zweite Kamera 12 den ersten Punkt 30 entlang eines zweiten Sehstrahl 32 und die dritte Kamera 13 diesen Punkt entlang eines dritten Sehstrahls 33. Das von der Oberfläche des Glases 2 reflektierte Licht wird daher von der ersten Kamera 11 unter einem ersten Betrachtungswinkel erfasst, den der Sehstrahl 31 mit der Senkrechten S in diesem Punkt 30 ausbildet, die zweite Kamera unter einem zweiten Betrachtungswinkel, den der Sehstrahl 32 mit der Senkrechten S in diesem Punkt 30 ausbildet, und die dritte Kamera 13 unter einem dritten Betrachtungswinkel, den der Sehstrahl 33 mit der Senkrechten S in diesem Punkt 30 ausbildet.

Entsprechend wird das von einem zweiten Punkt 40 der Inspektionslinie 1 reflektierte Licht durch die erste Kamera 11 unter einem Betrachtungswinkel erfasst, den ein erster Sehstrahl 41 mit der Senkrechten S in diesem Punkt 40 ausbildet, die zweite Kamera 12 unter einem zweiten Betrachtungswinkel, den der zweite Sehstrahl 42 mit der Senkrechten S in diesem Punkt 40 ausbildet und die dritte Kamera 13 unter einem zweiten Betrachtungswinkel, den der dritte Sehstrahl 43 mit der Senkrechten S in diesem Punkt 40 ausbildet.

Die von den Kameras 11, 12, 13 ermittelten Farbwerte für alle Punkte der Inspektionslinie 1 werden an die nicht dargestellte Auswerteeinheit übermittelt. Diese vergleicht die zu den jeweiligen Punkten ermittelten Farbwerte, das heißt beispielsweise die zu dem ersten Punkt 30 und zu dem zweiten Punkt 40 ermittelten Farbwerte mit vorgegebenen Farbsollwerten beziehungsweise Farbsollwert-Bereichen, wobei ein solcher Farbsollwert-Bereich beispielsweise einen bestimmten Blau- oder Grünbereich umfasst. Liegen die von den Kameras ermittelten Farbwerte für alle Punkte der Inspektionslinie in dem vorgegebenen Farbsollwert-Bereich, so ist das beschichtete Glas 2 fehlerfrei. Werden für mindestens einen Punkt der Inspektionslinie, für den zwei Farbwerte unter zwei verschiedenen Blickrichtungen erfasst werden, bei einem Vergleich dieser beiden Farbwerte für diesen Punkt mit dem vorgegebenen Farbsollwert oder dem vorgegebenen Farbsollwert-Bereich Abweichungen festgestellt, so liegt ein Fehlertyp 2 vor.

Weiter wird für mindestens einen konstanten Betrachtungswinkel analysiert, ob die ermittelten Farbwerte in einem, für den jeweiligen Betrachtungswinkel oder Betrachtungswinkel-Bereich vorgegebenen Farbsollwert oder Farbsollwert-Bereich liegen. Hierdurch kann ermittelt werden, ob ein Fehlertyp 1 vorliegt.

Vorzugsweise wird der Vergleich der ermittelten Farbwerte mit dem Farbsollwert oder dem Farbsollwert-Bereich im Lab-Farbraum durchgeführt. Hierfür werden die Farbwerte entweder bereits durch die jeweilige Kamera 11, 12, 13 in ein Wert nach dem Lab-Farbraum umgewandelt oder diese Umwandlung wird vor dem Vergleich durch die Auswerteeinrichtung vorgenommen.

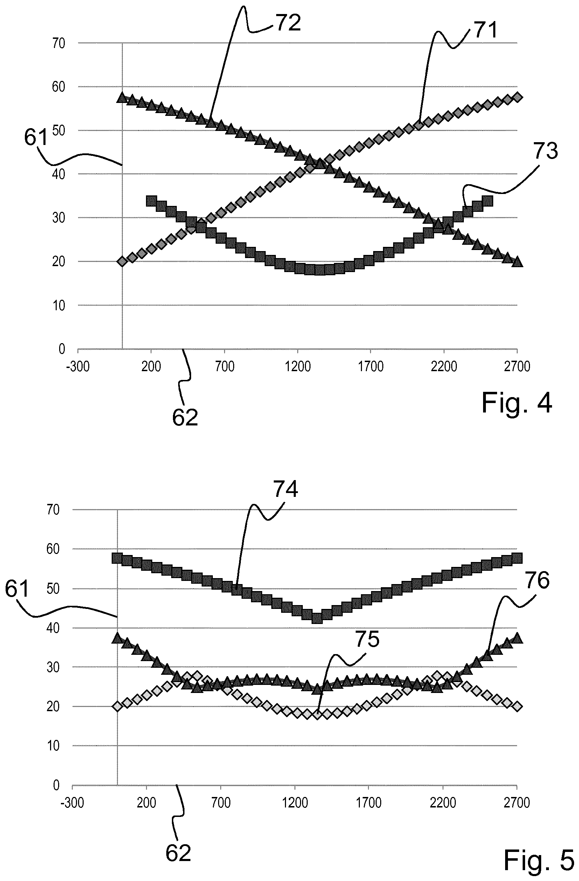

In Fig. 4 ist der Betrachtungswinkel der Kameras (Achse 61) in Abhängigkeit von der Position des Punkts auf der Inspektionslinie 1 (siehe Achse 62) dargestellt. Die Kurve 71 zeigt, dass sich der Betrachtungswinkel des reflektierten Lichts, das von den Punkten der Inspektionslinie 1 durch die erste Kamera 11 betrachtet wird, von einem Winkel von etwa 20° für einen Punkt auf der linken Seite der in Fig. 1 gezeigten Inspektionslinie 1 zu etwa 58° für einen Punkt auf der rechten Seite der Inspektionslinie 1 in Fig. 1 ändert. Entsprechend nimmt der Betrachtungswinkel für die Kamera 12 entlang der Inspektionslinie 1 von links nach rechts von etwa 58° bis auf 20° ab (siehe Kurve 72). Der von der dritten Kamera erfasste mittige Betrachtungswinkel-Bereich erstreckt sich zwischen etwa 18° und 35° (siehe Kurve 73).

Das Diagramm in Fig. 5 verdeutlicht die Auswertung anhand kleiner und großer Betrachtungswinkel und anhand der Betrachtungswinkeldifferenz. Hierfür werden die Betrachtungswinkel jeder Kamera 11, 12, 13 in jedem Punkt durch die Auswerteeinrichtung miteinander verglichen.

Die Kurve 74 zeigt die jeweils größten (maximalen) Betrachtungswinkel in jedem Punkt der Inspektionslinie 1, die zwischen etwa 58° und 42° liegen, und die in dem linken Teilbereich durch die zweite Kamera (siehe Kurve 72 in Fig. 4) und in dem rechten Teilbereich durch die erste Kamera (siehe Kurve 71 in Fig. 4) erzeugt werden. Die in Kurve 75 der Fig. 5 dargestellten kleinsten (minimalen) Betrachtungswinkel in jedem Punkt der Inspektionslinie 1 werden in einem ersten linken Bereich durch die erste Kamera (siehe Kurve 71 in Fig. 4) in einem mittigen Bereich durch die dritte Kamera (siehe Kurve 73 in Fig. 4) und in einem rechten Bereich durch die zweite Kamera gebildet. Um einen möglichst großen Betrachtungswinkelbereich abzudecken, werden jeweils die Farbwerte der zu dem jeweiligen Betrachtungswinkel gehörenden Kamera für jeden Punkt der Inspektionslinie 1, die den in Kurve 74 dargestellten größten Betrachtungswinkel bilden, mit dem entsprechenden, vorgegebenen Farbsollwert oder dem Farbsollwert-Bereich verglichen. Analog werden die Farbwerte der zu dem jeweiligen Betrachtungswinkel gehörenden Kamera für jeden Punkt der Inspektionslinie 1, die den in Kurve 75 dargestellten kleinsten Betrachtungswinkel bilden, mit dem entsprechenden, vorgegebenen Farbsollwert oder Farbsollwert-Bereich verglichen.

In einem weiteren Ausführungsbeispiel wird für jeden Punkt der Inspektionslinie 1 die Betrachtungswinkeldifferenz zwischen dem jeweiligen in Kurve 74 dargestellten größten Betrachtungswinkel und dem jeweiligen in Kurve 75 dargestellten kleinsten Betrachtungswinkel gebildet. Dies ergibt die Kurve 76 in Fig. 5. Ferner wird die Differenz aus den zugehörigen Farbwerten der jeweiligen Kameras, das heißt z.B. in einem linken Bereich der Inspektionslinie 1 zwischen den Farbwerten der zweiten Kamera und der ersten Kamera, in einem mittleren Bereich zunächst zwischen den Farbwerten der zweiten Kamera und der dritten Kamera und dann zwischen den Farbwerten der ersten Kamera und der dritten Kamera und einem rechten Bereich zwischen Farbwerten der ersten Kamera und der zweiten Kamera ermittelt. Diese werden dann (gegebenenfalls unter Einbeziehung der jeweiligen Betrachtungswinkeldifferenz, die in Kurve 76 dargestellt ist) dahingehend untersucht, ob die Farbwertdifferenzen in einem vorgegebenen Farbwertdifferenz-Sollwertbereich liegen.

Wird an einem Punkt oder mehreren Punkten der Inspektionslinie eine Abweichung der Differenz zwischen dem jeweiligen Farbwert für den größten Betrachtungswinkel und dem jeweiligen Farbwert für den kleinsten Betrachtungswinkel zu dem Farbwertdifferenz-Sollwert oder Farbwertdifferenz-Sollwertbereich erkannt, ergibt sich, dass ein Fehlertyp 2 vorliegt.

Wenn nun untersucht werden soll, ob Fehlertyp 1 vorliegt, muss der Farbwert für gleiche Betrachtungswinkel der ersten Kamera, der zweiten Kamera und der dritten Kamera bestimmt werden. Für einen bestimmten Betrachtungswinkel kann die Farbinspektion für Punkte der Inspektionslinie erfolgen, an denen z.B. die Kurven 74 und 75 in Fig. 5 durch horizontale Geraden geschnitten werden. Die Farbinspektion kann dabei für mehrere Sets von gleichen Betrachtungswinkeln, die sich über die gesamte Breite der Inspektionslinie erstrecken, durchgeführt werden, was durch eine Parallelverschiebung der Schnittgeraden veranschaulicht werden kann. Durch einen Vergleich der für die Sets von gleichen Betrachtungswinkeln ermittelten Farbwerte mit dem bestimmten, vorgegebenen Farbsollwert oder dem bestimmten, vorgegebenen Farbsollwertbereich kann über die gesamte Breite der Inspektionslinie festgestellt werden, ob ein Fehlertyp 1 vorliegt.

Das, was über die Kurve 75 gesagt wurde, gilt mit Beschränkung auch für die Kurve 74. Allerdings ist bei den in Kurve 74 dargestellten Betrachtungswinkeln der Winkelbereich größer und es werden nur zwei Kameras herangezogen.

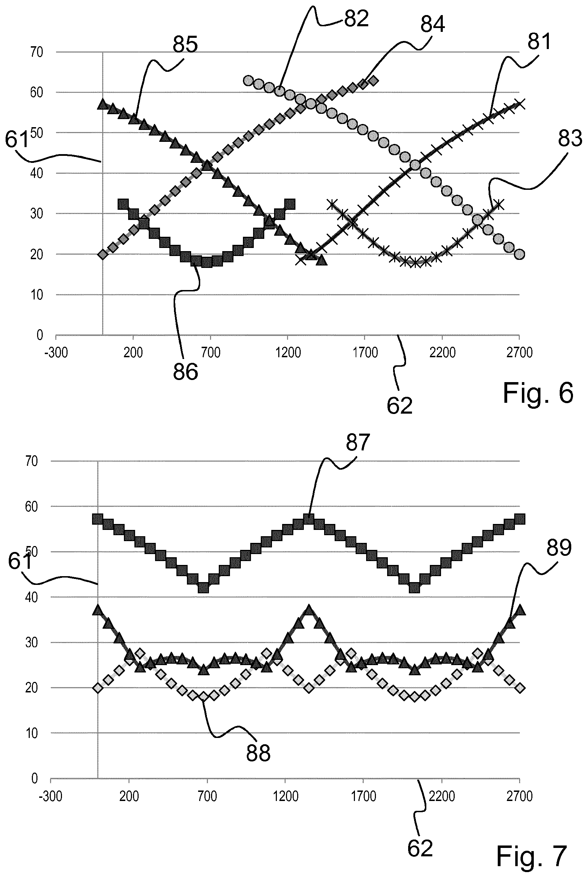

Fig. 3 zeigt ein zweites Ausführungsbeispiel einer erfindungsgemäßen Vorrichtung mit einer ersten Kamera 11', einer zweiten Kamera 12', einer dritten Kamera 13', einer vierten Kamera 14', einer fünften Kamera 15' und einer sechsten Kamera 16', die nebeneinander auf einem Träger 5 angeordnet sind und das von der nicht dargestellten, linienförmigen und weißen Lichtquelle ausgestrahlte, von der Oberfläche des beschichteten Glases 2 reflektierte Licht entlang einer Inspektionslinie 1' erfassen. Jede Kamera ist um einen Winkel zu der Senkrechten auf der Inspektionslinie geneigt, beispielsweise die erste Kamera 11' um einen Winkel α1', die zweite Kamera 12' um einen zweiten Winkel α2', die vierte Kamera 14' um einen Winkel α4' und die fünfte Kamera 15' um einen fünften Winkel α5'. Die Neigungswinkel der dritten Kamera 13' und der sechsten Kamera 16' sind nicht eingezeichnet. Analog zu dem ersten Ausführungsbeispiel weisen alle Kameras 11', 12', 13', 14', 15', 16' große Öffnungswinkel auf, so dass sie das reflektierte Licht eines großen Bereichs der Inspektionslinie 1' unter verschiedenen Betrachtungswinkeln erfassen können. Der erfasste Betrachtungswinkelbereich ist ortsabhängig in dem in Fig. 6 dargestellten Diagramm zu entnehmen, wobei der Betrachtungswinkelbereich der ersten Kamera 11' anhand der Kurve 81, der Betrachtungswinkelbereich der zweiten Kamera 12' anhand der Kurve 82, der Betrachtungswinkelbereich der dritten Kamera 13' anhand der Kurve 83, der Betrachtungswinkelbereich der vierten Kamera 14' anhand der Kurve 84, der Betrachtungswinkelbereich der fünften Kamera 15' anhand der Kurve 85 und der Betrachtungswinkelbereich der sechsten Kamera 16' anhand der Kurve 86 veranschaulicht ist. Analog zu dem Vorgehen, das anhand des in Fig. 5 gezeigten Diagramms oben erläutert wurde, können auch bei dieser erfindungsgemäßen Vorrichtung jeweils punktweise lediglich die Farbwerte der Kamera für den Vergleich mit einem Farbsollwert oder Farbsollwert-Bereich herangezogen werden, die für einen bestimmten Punkt der Inspektionslinie 1' einen großen Betrachtungswinkel (vgl. Kurve 87 in Fig. 7) und/oder einen kleinen Betrachtungswinkel (vgl. Kurve 88 in Fig. 7) aufweisen. Analog zu dem oben beschriebenen Vorgehen kann auch lediglich eine Differenz der Farbwerte für die in Kurve 87 gezeigten großen Betrachtungswinkel und die in Kurve 88 gezeigten kleinen Betrachtungswinkel (siehe Kurve 89 in Fig. 7) zum Vergleich mit einem entsprechenden Farbwertdifferenz-Sollwertbereich herangezogen werden.

Auf dem Träger der in Fig. 3 dargestellten erfindungsgemäßen Vorrichtung können weitere Messeinrichtungen 50 angeordnet sein, welche das Glas 2 in Bezug auf weitere Fehler untersuchen, wie z.B. optische Fehler, Blasen, Einschlüsse, Kratzer. Hierfür werden vorzugsweise hochauflösende Kameras eingesetzt.

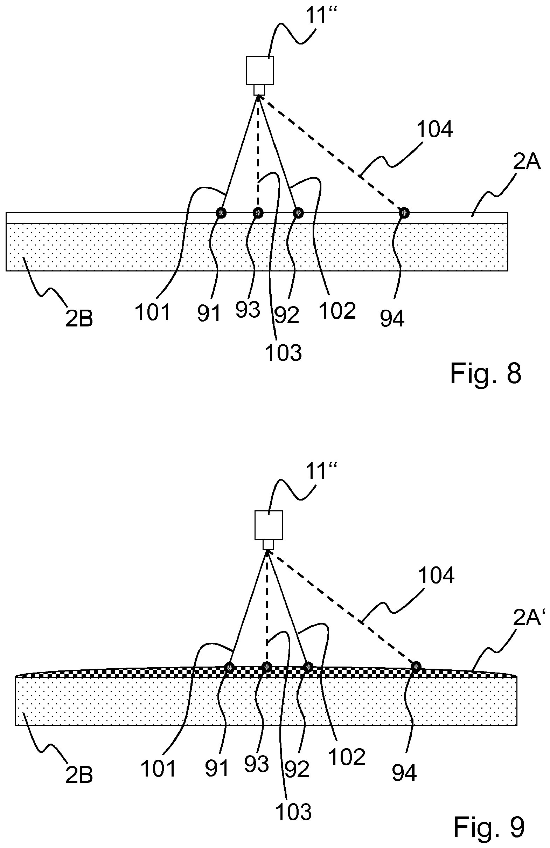

Im Folgenden soll anhand der Figuren 8 bis 10 dargestellt werden, welche Aussagen über die Qualität einer Beschichtung 2A eines Glasmaterials 2B mit einer erfindungsgemäßen Vorrichtung mit einer einzigen Kamera 11" noch getroffen werden können.

Die Kamera 11" steht senkrecht oberhalb des mit der Beschichtung 2A, 2A', 2A" versehenen Glasmaterials 2B und der an der Oberfläche der Beschichtung 2A, 2A', 2A" verlaufenden, nicht dargestellten Inspektionslinie mit unter Anderem den Punkten 91, 92, 93, 94. Die Kamera 11" weist einen so großen Öffnungswinkel auf, dass das mit der Beschichtung versehene Glasmaterial in seiner gesamten Breite durch die Kamera 11" untersucht werden kann. Die nicht dargestellte Lichtquelle (siehe Erläuterungen zu Fig. 2) ist auch oberhalb des beschichteten Glasmaterials, schräg zur Kamera 11" angeordnet.

Die Verhältnisse sind am einfachsten, wenn die Kamera 11", wie in den Fig. 8 bis 10 dargestellt, senkrecht auf das beschichtete Glasmaterial schaut. Im Einzelnen beobachtet die Kamera 11" beispielsweise das an den Punkten 91, 92 der Inspektionslinie reflektierte Licht der Lichtquelle unter dem gleichen Betrachtungswinkel entlang der Sehstrahlen 101 und 102. Das an den Punkten 93 und 94 reflektierte Licht der Lichtquelle wird entlang der Sehstrahlen 103 und 104 unter verschiedenem Betrachtungswinkel erfasst.

Die in Fig. 8 dargestellte Beschichtung 2A ist gleichmäßig dick, so dass der Fehlertyp 1 nicht vorliegt. Bei gleicher Schichtdicke und bei gleichen Betrachtungswinkeln (siehe Punkte 91, 92 und Sehstrahlen 101 und 102), sind die Farbwerte gleich. Bei unterschiedlichen Betrachtungswinkeln (siehe Punkte 92, 93, 94 und Sehstrahlen 102, 103, 104) können die Farbwerte unterschiedlich sein. Es ist nicht eindeutig feststellbar, ob Fehlertyp 1 oder 2 vorliegt. Es kann lediglich eine Farbabweichung von dem vorgegebenen Sollwert oder Sollwertbereich festgestellt werden. Insbesondere ist eine ganzflächige Untersuchung hinsichtlich des Fehlertyps 2, wobei für jede Stelle des beschichteten Glases jeweils mindestens zwei Farbwerte für verschiedene Betrachtungswinkel erfasst werden, wie dies mit den in Fig. 1 und 3 dargestellten Ausführungsbeispielen bewerkstelligt werden kann, nicht möglich.

Die folgende Fig. 9 zeigt eine ungleichmäßige Beschichtung 2A' symmetrisch zur optischen Achse der Kamera 11".

Bei dieser Variante einer Beschichtung werden bei gleichen Betrachtungswinkeln (siehe Punkte 91, 92 und Sehstrahlen 101 und 102) gleiche Farbwerte ermittelt, obwohl Fehlertyp 1 vorliegt. Bei unterschiedlichen Betrachtungswinkeln (siehe Punkte 92, 93, 94 und Sehstrahlen 102, 103, 104) werden unterschiedliche Farbwerte detektiert. Fehlertyp 2 kann analog zu Fig. 8 für zwei separate Stellen unabhängig von dessen Ursache wahrgenommen werden.

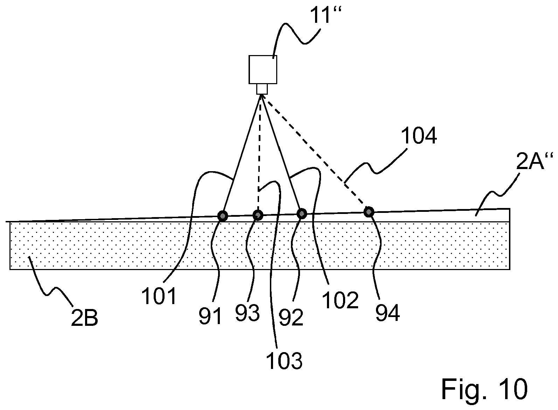

Es soll noch der Fall einer keilförmigen Beschichtung 2A" betrachtet werden, welche in Fig. 10 dargestellt ist.

Bei gleichen Betrachtungswinkeln (siehe Punkte 91, 92 und Sehstrahlen 101 und 102) werden unterschiedliche Farbwerte ermittelt und bei unterschiedlichen Betrachtungswinkeln (siehe Punkte 92, 93, 94 und Sehstrahlen 102, 103, 104) werden erst recht unterschiedliche Farbwerte erfasst. Auch bei dieser Beschichtungsvariante kann Fehlertyp 2 jeweils für zwei separate Stellen analog zu Fig. 8 über die gesamte Breite der Inspektionslinie unabhängig von dessen Ursache wahrgenommen werden.

Insgesamt ist die Feststellung einer Farbabweichung unter verschiedenen Betrachtungswinkeln zunächst unabhängig von Fehlertyp 1 oder 2. Bei großen Farbabweichungen über die Inspektionslinie oder die Fläche des zu untersuchenden beschichteten Materials ist dieses auf jeden Fall z.B. für eine Fassade ungeeignet.

Eine Farbabweichung unabhängig vom Fehlertyp kann bereits mit einer Kamera mit entsprechendem Öffnungswinkel festgestellt werden. Ein solches Ergebnis ist für viele praktische Fälle ausreichend. Zum Ausschluss von Fehlertyp 1 müssen jedoch mindestens zwei Kameras eingesetzt werden, welche auf unterschiedliche Stellen des Glases mit dem gleichen Betrachtungswinkel blicken und Farbwertgleichheit feststellen.

### Bezugszeichenliste

- 1, 1': Inspektionslinie
- 2: Glas mit Beschichtung auf der Oberfläche
- 2A, 2A', 2A": Beschichtung
- 2B: Material (Glas)
- 3: Pfeil (Bewegungsrichtung)
- 5: Träger
- 11, 11', 11": erste Kamera
- 12, 12': zweite Kamera
- 13, 13': dritte Kamera
- 14': vierte Kamera
- 15': fünfte Kamera
- 16': sechste Kamera
- 18: Lichtquelle
- 21: erster Öffnungswinkel der ersten Kamera 11
- 22: zweiter Öffnungswinkel der zweiten Kamera 12
- 23: dritter Öffnungswinkel der dritten Kamera 13
- 31, 32, 33: Sehstrahl
- 41, 42, 43: Sehstrahl
- 30, 40: Punkt auf Inspektionslinie 1
- 50: weitere Messeinrichtung
- 61: Achse (Betrachtungswinkel in Grad)
- 62: Achse (Position des Punktes auf der Inspektionslinie 1, 1' in Millimetern)
- 71, 72, 73, 74, 75, 76: Kurve
- 81, 82, 83, 84, 85, 86, 87, 88, 89: Kurve
- 91, 92, 93, 94: Punkt auf Inspektionslinie
- 101, 102, 103, 104: Sehstrahl
- α1, α1': Neigungswinkel der ersten Kamera 11, 11' zur Senkrechten auf der Oberfläche
- α2': Neigungswinkel der zweiten Kamera 12' zur Senkrechten auf der Oberfläche
- α4': Neigungswinkel der vierten Kamera 14' zur Senkrechten auf der Oberfläche
- α5': Neigungswinkel der fünften Kamer 15' zur Senkrechten auf der Oberfläche
- b: Breite der Inspektionslinie 1
- S: Senkrechte auf der Oberfläche des Glases 2

## Patentansprüche

1. Vorrichtung zur Inspektion eines mit einer beschichteten Oberfläche versehenen Materials (2), vorzugsweise Glas, mit
einer über der Oberfläche angeordneten Lichtquelle (18), welche Licht in einem vorgegebenen Wellenlängenbereich in Richtung der Oberfläche abgibt,
und einer ersten Kamera (11, 11', 11", 14'), wobei die erste Kamera (11, 11', 11", 14') zur Ermittlung eines Farbwerts des erfassten Lichts eingerichtet ist, wobei die erste Kamera (11, 11', 11", 14') oberhalb einer auf der Oberfläche liegenden Inspektionslinie derart geneigt zur Lichtquelle angeordnet ist, dass sie die spiegelnde Reflexion des von der Lichtquelle ausgesandten Lichts an der beschichteten Oberfläche im Bereich der Inspektionslinie sieht, und einen ersten Öffnungswinkel (21) besitzt derart,
dass von einem ersten Punkt der Inspektionslinie reflektiertes Licht der Lichtquelle (18) unter einem ersten Betrachtungswinkel und von einem zu dem ersten Punkt beabstandeten zweiten Punkt der Inspektionslinie unter einem zweiten Betrachtungswinkel separat erfassbar ist, wobei der erste Betrachtungswinkel und der zweite Betrachtungswinkel verschieden sind, wobei die erste Kamera (11, 11', 11", 14') derart eingerichtet ist, dass sie einen ersten Farbwert des von dem ersten Punkt reflektierten Lichts und einen zweiten Farbwert des von dem zweiten Punkt reflektierten Lichts ermittelt,
wobei eine mit der ersten Kamera (11, 11', 11", 14') verbundene Auswerteeinrichtung vorgesehen ist, welche zum Vergleich des ersten Farbwerts und des zweiten Farbwerts jeweils mit einem bestimmten, vorgegebenen Farbsollwert oder mit einem bestimmten, vorgegebenen Farbsollwert-Bereich oder zum Vergleich einer Differenz des ersten Farbwerts und des zweiten Farbwerts mit einem bestimmten, vorgegebenen Farbwertdifferenz-Sollwert oder mit einem bestimmten, vorgegebenen Farbwertdifferenz-Sollwertbereich eingerichtet ist, wobei mindestens eine zweite Kamera (12, 12', 15') vorgesehen ist, wobei die zweite Kamera (12, 12', 15') ebenfalls zur Ermittlung eines Farbwerts des erfassten Lichts eingerichtet ist,
wobei die zweite Kamera (12, 12', 15') oberhalb der auf der Oberfläche liegenden Inspektionslinie und geneigt zur Lichtquelle angeordnet ist, wobei die zweite Kamera (12, 12', 15') einen zweiten Öffnungswinkel (22) besitzt derart, dass von jedem Punkt der Inspektionslinie reflektiertes Licht der Lichtquelle (18) vorzugsweise gleichzeitig durch die erste Kamera (11, 11', 11", 14') und durch die zweite Kamera (12, 12', 15') erfassbar ist, wobei die erste Kamera (11, 11', 11", 14') und die zweite Kamera (12, 12', 15') derart eingerichtet sind, dass sie jeweils den Farbwert des von jedem Punkt der Inspektionslinie reflektierten Lichts ermitteln, wobei die mit der ersten Kamera und der zweiten Kamera verbundene Auswerteeinrichtung derart eingerichtet ist, dass sie die Farbwerte der ersten Kamera und der zweiten Kamera zu jedem Punkt der Inspektionslinie zu einem Vergleich mit dem bestimmten, vorgegebenen Farbsollwert oder dem bestimmten, vorgegebenen Farbsollwert-Bereich heranzieht, wobei die Auswerteeinrichtung weiter derart eingerichtet ist, dass sie den Farbwert des reflektierten Lichts von verschiedenen Punkten der Inspektionslinie zusätzlich abhängig von deren zugehörigen Betrachtungswinkel auswertet.

2. Vorrichtung nach Anspruch 1, **dadurch gekennzeichnet, dass** die Auswerteeinrichtung derart eingerichtet ist, dass die Differenz des ersten Farbwerts des ersten Punktes der Inspektionslinie und des zweiten Farbwerts des zweiten Punktes der Inspektionslinie mit dem bestimmten, vorgegebenen Farbsollwert oder dem bestimmten, vorgegebenen Farbsollwert-Bereich verglichen werden, wobei der erste Punkt und der zweite Punkt durch die Kamera in verschiedene Richtungen entlang der Inspektionslinie unter dem gleichen oder annähernd dem gleichen Betrachtungswinkel beobachtet werden.

3. Vorrichtung nach einem der vorhergehenden Ansprüche, **dadurch gekennzeichnet, dass** die erste Kamera (11, 11', 11", 14') und die mindestens eine zweite Kamera (12, 12', 15') auf einem gemeinsamen Träger (5) angeordnet sind.

4. Vorrichtung nach einem der vorhergehenden Ansprüche, **dadurch gekennzeichnet, dass** die erste Kamera (11, 11', 11", 14') und/oder die zweite Kamera (12, 12', 15') jeweils eine Zeilenkamera oder eine Flächenkamera ist.

5. Vorrichtung nach einem der vorhergehenden Ansprüche, **dadurch gekennzeichnet, dass** sich der erste Betrachtungswinkel und der zweite Betrachtungswinkel um mindestens 10°, vorzugsweise um mindestens 15°, besonders bevorzugt um mindestens 20° unterscheiden.

6. Vorrichtung nach einem der vorhergehenden Ansprüche, **dadurch gekennzeichnet, dass** das Material (2) relativ zu der ersten Kamera (11, 11', 11", 14') und der zweiten Kamera (12, 12', 15') und zu der Lichtquelle (18) in Richtung einer Vorschubrichtung (3) bewegbar ist, wobei vorzugsweise die Lichtquelle (18) und/oder die erste Kamera (11, 11', 11", 14') und/oder die zweite Kamera (12, 12', 15') in Vorschubrichtung oder gegen die Vorschubrichtung geneigt sind.

7. Vorrichtung nach einem der vorhergehenden Ansprüche, **dadurch gekennzeichnet, dass** mindestens eine dritte Kamera (13, 13', 16') vorgesehen ist, welche zur Ermittlung eines Farbwerts des erfassten Lichts eingerichtet ist, wobei die dritte Kamera (13, 13', 16') oberhalb der Inspektionslinie und geneigt zur Lichtquelle (18) angeordnet ist, wobei die dritte Kamera einen dritten Öffnungswinkel besitzt derart, dass zumindest für die Punkte der Inspektionslinie in einem bestimmten Bereich der Inspektionslinie zusätzlich das reflektierte Licht der Lichtquelle (18) erfassbar und der zugehörige Farbwert ermittelbar ist, wobei die Auswerteeinrichtung für jeden Punkt des bestimmten Bereichs der Inspektionslinie den Betrachtungswinkel der dritten Kamera mit dem Betrachtungswinkel der ersten Kamera (11, 11', 11", 14') und/oder dem Betrachtungswinkel der zweiten Kamera (12, 12', 15') vergleicht und für den entsprechenden Punkt des bestimmten Bereichs diejenigen zwei Farbwerte der ersten Kamera (11, 11', 11", 14'), der zweiten Kamera (12, 12', 15') und der dritten Kamera (13, 13', 16') zum Vergleich mit dem bestimmten, vorgegebenen Farbsollwert oder dem bestimmten, vorgegebenen Farbsollwert-Bereich heranzieht, für welche die Differenz der zugehörigen Betrachtungswinkel am größten ist.

8. Vorrichtung nach einem der vorhergehenden Ansprüche, **dadurch gekennzeichnet, dass** die Auswerteeinrichtung in jedem Punkt der Inspektionslinie jeweils eine Farbwertdifferenz von zwei Farbwerten zweier bestimmter Kameras, die das reflektierte Licht dieses Punkts beobachten, ermittelt und diese Farbwertdifferenz mit einem bestimmten, vorgegebenen Farbwertdifferenz-Sollwert oder einem bestimmten, vorgegebenen Farbwertdifferenz-Sollwertbereich vergleicht.

9. Verfahren zur Inspektion eines mit einer beschichteten Oberflächen versehenen Materials (2), vorzugsweise Glas, wobei
eine über der Oberfläche angeordnete Lichtquelle (18), welche Licht in einem vorgegebenen Wellenlängenbereich in Richtung der Oberfläche abgibt,
und eine erste Kamera (11, 11', 11", 14') vorgesehen sind, wobei die erste Kamera (11, 11', 11", 14') zur Ermittlung eines Farbwerts des erfassten Lichts eingerichtet ist, wobei die erste Kamera (11, 11', 11", 14') oberhalb einer auf der Oberfläche liegenden Inspektionslinie derart geneigt zur Lichtquelle angeordnet ist, dass sie die spiegelnde Reflexion des von der Lichtquelle ausgesandten Lichts an der beschichteten Oberfläche im Bereich der Inspektionslinie sieht, und einen ersten Öffnungswinkel (21) besitzt, mit den folgenden Schritten:
• mittels der ersten Kamera (11, 11', 11", 14') wird das von einem ersten Punkt der Inspektionslinie reflektierte Licht der Lichtquelle (18) unter einem ersten Betrachtungswinkel und das von einem, zu dem ersten Punkt beabstandeten zweiten Punkt der Inspektionslinie unter einem zweiten Betrachtungswinkel separat erfasst, wobei der erste Betrachtungswinkel und der zweite Betrachtungswinkel verschieden sind, wobei die erste Kamera einen ersten Farbwert des von dem ersten Punkt reflektierten Lichts und einen zweiten Farbwert des von dem zweiten Punkt reflektierten Lichts ermittelt,
• wobei mittels einer mit der ersten Kamera (11, 11', 11", 14') verbundenen Auswerteeinrichtung der erste Farbwert und der zweite Farbwert jeweils mit einem bestimmten, vorgegebenen Farbsollwert oder mit einem bestimmten, vorgegebenen Farbsollwert-Bereich oder eine Differenz des ersten Farbwerts und des zweiten Farbwerts mit einem bestimmten, vorgegebenen Farbwertdifferenz-Sollwert oder mit einem bestimmten, vorgegebenen Farbwertdifferenz-Sollwertbereich verglichen werden,
wobei mindestens eine zweite Kamera (12, 12', 15') vorgesehen ist, wobei die zweite Kamera (12, 12', 15') ebenfalls zur Ermittlung eines Farbwerts des erfassten Lichts eingerichtet ist,
wobei die zweite Kamera (12, 12', 15') oberhalb der auf der Oberfläche liegenden Inspektionslinie und geneigt zur Lichtquelle angeordnet ist, wobei die zweite Kamera (12, 12', 15') einen zweiten Öffnungswinkel (22) besitzt, wobei durch die erste Kamera (11, 11', 11", 14') und durch die zweite Kamera (12, 12', 15') von jedem Punkt der Inspektionslinie reflektiertes Licht der Lichtquelle (18) vorzugsweise gleichzeitig erfasst und jeweils der Farbwert des von jedem Punkt der Inspektionslinie reflektierten Lichts ermittelt wird sowie durch die Auswerteeinrichtung mit dem bestimmten, vorgegebenen Farbsollwert oder dem bestimmten, vorgegebenen Farbsollwert-Bereich verglichen wird, wobei mittels der Auswerteeinrichtung der Farbwert des reflektierten Lichts von verschiedenen Punkten der Inspektionslinie zusätzlich abhängig von deren zugehörigen Betrachtungswinkel ausgewertet wird.

10. Verfahren nach Anspruch 9, **dadurch gekennzeichnet, dass** mindestens eine dritte Kamera (13, 13', 16') vorgesehen ist, welche zur Ermittlung eines Farbwerts des erfassten Lichts eingerichtet ist, wobei die dritte Kamera (13, 13', 16') oberhalb der Inspektionslinie und geneigt zur Lichtquelle (18) angeordnet ist, wobei die dritte Kamera (13, 13', 16') einen dritten Öffnungswinkel (23) besitzt, wobei durch die dritte Kamera (13, 13', 16') zumindest für die Punkte der Inspektionslinie in einem bestimmten Bereich der Inspektionslinie zusätzlich das reflektierte Licht der Lichtquelle (18) erfasst und der zugehörige Farbwert ermittelt wird, wobei durch die Auswerteeinrichtung für jeden Punkt des bestimmten Bereichs der Inspektionslinie der Betrachtungswinkel der dritten Kamera (13, 13', 16') mit dem Betrachtungswinkel der ersten Kamera (11, 11', 11", 14') und/oder dem Betrachtungswinkel der zweiten Kamera (12, 12', 15') verglichen wird und für den entsprechenden Punkt des bestimmten Bereichs diejenigen zwei Farbwerte der ersten Kamera (11, 11', 11", 14'), der zweiten Kamera (12, 12', 15') und der dritten Kamera (13, 13', 16') zum Vergleich mit dem bestimmten, vorgegebenen Farbsollwert oder dem bestimmten, vorgegebenen Farbsollwert-Bereich herangezogen werden, für welche die Differenz der zugehörigen Betrachtungswinkel am größten ist.

11. Verfahren nach einem der Ansprüche 9 bis 10, **dadurch gekennzeichnet, dass** mittels der Auswerteeinrichtung in jedem Punkt der Inspektionslinie jeweils eine Farbwertdifferenz von zwei Farbwerten zweier bestimmter Kameras, die das reflektierte Licht dieses Punkts beobachten, ermittelt und diese Farbwertdifferenz mit einem bestimmten, vorgegebenen Farbwertdifferenz-Sollwert oder einem bestimmten, vorgegebenen Farbwertdifferenz-Sollwertbereich verglichen wird.

## Claims

1. Apparatus for inspecting a material (2) provided with a coated surface, preferably glass, with
a light source (18) disposed above the surface, which emits light in a predetermined wavelength range in the direction of the surface,
and a first camera (11, 11', 11", 14'), wherein the first camera (11, 11', 11", 14') is configured to determine a color value of the detected light,
wherein the first camera (11, 11', 11", 14') is disposed above an inspection line located on the surface inclined to the light source in such a way that it sees the specular reflection of the light emitted by the light source from the coated surface in the region of the inspection line, and has a first aperture angle (21) in such a way, that light of the light source (18) reflected from a first point of the inspection line is separately detectable at a first viewing angle and from a second point of the inspection line spaced from the first point is separately detectable at a second viewing angle, wherein the first viewing angle and the second viewing angle are different, wherein the first camera (11, 11', 11", 14') is configured such that it determines a first color value of the light reflected from the first point and a second color value of the light reflected from the second point,
wherein an evaluation device connected to the first camera (11, 11', 11", 14') is provided, which is configured to compare the first color value and the second color value in each case with a specific, predetermined color setpoint value or with a specific, predetermined color setpoint value range or to compare a difference of the first color value and the second color value with a specific, predetermined color value difference setpoint value or with a specific, predetermined color value difference setpoint value range, predetermined color value difference setpoint value or with a specific, predetermined color value difference setpoint value range, wherein the at least one second camera (12, 12', 15') being provided, the second camera (12, 12', 15') likewise is configured to determine a color value of the detected light,
wherein the second camera (12, 12', 15') is disposed above the inspection line located on the surface and inclined to the light source, wherein the second camera (12, 12', 15') has a second aperture angle (22) such that light of the light source (18) reflected from each point of the inspection line is detectable, preferably simultaneously, by the first camera (11, 11', 11", 14') and by the second camera (12, 12', 15'), wherein the first camera (11, 11', 11", 14') and the second camera (12, 12', 15') are configured such that they each determine the color value of the light reflected from each point of the inspection line, wherein the evaluation device connected to the first camera and the second camera is configured such that it uses the color values of the first camera and of the second camera at each point of the inspection line for a comparison with the specific, predetermined color setpoint or the specific, predetermined color setpoint range, the evaluation device is further configured such that it evaluates the color value of the reflected light from different points of the inspection line additionally as a function of their associated viewing angle.

2. The apparatus according to claim 1, **characterized in that** the evaluation device is configured such that the difference of the first color value of the first point of the inspection line and the second color value of the second point of the inspection line are compared with the specific, predetermined color setpoint or the specific, predetermined color setpoint range, wherein the first point and the second point are observed by the camera in different directions along the inspection line under the same or approximately the same viewing angle.

3. The apparatus according to any one of the preceding claims, **characterized in that** the first camera (11, 11', 11", 14') and the at least one second camera (12, 12', 15') are disposed on a common carrier (5).

4. The apparatus according to any one of the preceding claims, **characterized in that** the first camera (11, 11', 11", 14') and/or the second camera (12, 12', 15') are either a line scan camera or an area scan camera.

5. The apparatus according to any one of the preceding claims, **characterized in that** the first viewing angle and the second viewing angle differ by at least 10°, preferably by at least 15°, more preferably by at least 20°.

6. The apparatus according to any one of the preceding claims, **characterized in that** the material (2) is movable relative to the first camera (11, 11', 11", 14') and the second camera (12, 12', 15') and to the light source (18) into the direction of a feed direction (3), wherein preferably the light source (18) and/or the first camera (11, 11', 11", 14') and/or the second camera (12, 12', 15') being inclined in the feed direction or against the feed direction.

7. The apparatus according to any one of the preceding claims, **characterized in that** at least one third camera (13, 13', 16') is provided, which is set up to determine a color value of the detected light, the third camera (13, 13', 16') being disposed above the inspection line and inclined to the light source (18), wherein the third camera has a third aperture angle such that, at least for the points of the inspection line in a specific section of the inspection line, the reflected light of the light source (18) is additionally be detectable and the associated color value determinable,
wherein the evaluation device compares for each point of the specific section of the inspection line the viewing angle of the third camera with the viewing angle of the first camera (11, 11', 11", 14') and/or the viewing angle of the second camera (12, 12', 15') and uses for the corresponding point of the specific section those two color values of the first camera (11, 11', 11", 14'), of the second camera (12, 12', 15') and of the third camera (13, 13', 16') for comparison with the specific, predetermined color setpoint value or the specific, predetermined color setpoint value range, for which the difference of the associated viewing angles is greatest.

8. The apparatus according to any one of the preceding claims, **characterized in that** the evaluation device determines for each point of the inspection line in each case a color value difference of two color values of two specific cameras which observe the reflected light of this point, and compares this color value difference with a specific, predetermined color value difference setpoint or a specific, predetermined color value difference setpoint range.

9. Method for inspecting a material (2) provided with a coated surface, preferably glass, wherein
a light source (18) disposed above the surface, which emits light in a predetermined wavelength range in the direction of the surface,
and a first camera (11, 11', 11", 14') are provided, the first camera (11, 11', 11", 14') is configured to determine a color value of the detected light, the first camera (11, 11', 11", 14') is disposed above an inspection line located on the surface in such a way inclined to the light source that it sees the specular reflection of the light emitted by the light source from the coated surface in the region of the inspection line, and has a first aperture angle (21), having the following steps:
• by means of the first camera (11, 11', 11", 14'), the light of the light source (18) reflected from a first point of the inspection line is separately detected at a first viewing angle and the light reflected from a second point of the inspection line spaced from the first point is separately detected at a second viewing angle, wherein the first viewing angle and the second viewing angle are different, wherein the first camera determines a first color value of the light reflected from the first point and a second color value of the light reflected from the second point,
• wherein, by means of an evaluation device connected to the first camera (11, 11', 11", 14'), the first color value and the second color value are each compared with a specific, predetermined color setpoint or with a specific, predetermined color setpoint range, or a difference of the first color value and the second color value is compared with a specific, predetermined color value difference setpoint or with a specific, predetermined color value difference setpoint range,
wherein at least one second camera (12, 12', 15') is provided, wherein the second camera (12, 12', 15') is also configured to determine a color value of the detected light,
wherein the second camera (12, 12', 15') is disposed above the inspection line located on the surface and inclined to the light source, wherein the second camera (12, 12', 15') has a second aperture angle (22), wherein light of the light source (18) reflected from each point of the inspection line is detected preferably simultaneously by the first camera (11, 11', 11", 14') and by the second camera (12, 12', 15') of the light source (18) reflected from each point of the inspection line are detected preferably simultaneously and in each case the color value of the light reflected from each point of the inspection line is determined and compared by the evaluation device with the specific, predetermined color setpoint or the specific, predetermined color setpoint range, wherein the color value of the reflected light from different points of the inspection line being additionally evaluated by means of the evaluation device as a function of their associated viewing angle.

10. Method according to claim 9, **characterized in that** at least one third camera (13, 13', 16') is provided, which is configured to determine a color value of the detected light, the third camera (13, 13', 16') being dispose above the inspection line and inclined to the light source (18), wherein the third camera (13, 13', 16') has a third aperture angle (23), wherein the reflected light of the light source (18) is additionally detected by the third camera (13, 13', 16') at least for the points of the inspection line in a specific section of the inspection line and the associated color value is determined, wherein, for each point of the specific section of the inspection line, the evaluation device compares the viewing angle of the third camera (13, 13', 16') with the viewing angle of the first camera (11, 11', 11", 14') and/or the viewing angle of the second camera (12, 12', 15') and, for the corresponding point of the specific section, those two color values of the first camera (11, 11', 11", 14'), of the second camera (12, 12', 15') and of the third camera (13, 13', 16') are used for comparison with the specific, predetermined color setpoint value or the specific, predetermined color setpoint value range for which the difference of the associated viewing angles is greatest.

11. Method according to any one of the claims 9 to 10, **characterized in that** by means of the evaluation device in each point of the inspection line in each case a color value difference of two color values of two specific cameras, which observe the reflected light of this point, is determined and this color value difference is compared with a specific, predetermined color value difference setpoint or a specific, predetermined color value difference setpoint range.

## Revendications

1. Dispositif d'inspection d'un matériau (2) doté d'une surface revêtue, de préférence du verre, avec
une source lumineuse (18) disposée au-dessus de la surface, laquelle délivre de la lumière dans une plage de longueurs d'ondes prédéfinie en direction de la surface,
et une première caméra (11, 11', 11", 14'), sachant que la première caméra (11, 11', 11", 14') est agencée pour la détermination d'une valeur chromatique de la lumière saisie,
sachant que la première caméra (11, 11', 11", 14') est disposée au-dessus d'une ligne d'inspection située sur la surface inclinée vers la source lumineuse de telle manière qu'elle voit la réflexion réfléchissante de la lumière émise par la source lumineuse sur la surface revêtue dans le secteur de la ligne d'inspection et possède un premier angle d'ouverture (21) de telle manière,
qu'une lumière de la source lumineuse (18), réfléchie par un premier point de la ligne d'inspection peut être séparément saisie sous un premier angle d'observation et sous un deuxième angle d'observation par un deuxième point de la ligne d'observation distant du premier point, sachant que le premier angle d'observation et le deuxième angle d'observation sont différents, sachant que la première caméra (11, 11', 11", 14') est agencée de telle manière qu'elle détermine une première valeur chromatique de la lumière réfléchie par le premier point et une deuxième valeur chromatique de la lumière réfléchie par le deuxième point,
sachant qu'un système d'évaluation relié à la première caméra (11, 11', 11", 14') est prévu, lequel est agencé pour la comparaison de la première valeur chromatique et de la deuxième valeur chromatique respectivement à une valeur chromatique théorique déterminée, prédéfinie ou à une plage de valeurs chromatiques théoriques déterminée, prédéfinie ou pour la comparaison d'une différence de la première valeur chromatique et de la deuxième valeur chromatique à une valeur chromatique théorique de différence de valeur chromatique déterminée, prédéfinie ou à une plage de valeurs théoriques de différence de valeur chromatique déterminée, prédéfinie,
sachant qu'au moins une deuxième caméra (12, 12', 15') est prévue, sachant que la deuxième caméra (12, 12', 15') est également agencée pour la détermination d'une valeur chromatique de la lumière saisie,
sachant que la deuxième caméra (12, 12', 15') est disposée au-dessus de la ligne d'inspection située sur la surface et inclinée vers la source lumineuse, sachant que la deuxième caméra (12, 12', 15') possède un deuxième angle d'ouverture (22) de telle manière que la lumière de la source lumineuse (18), réfléchie par chaque point de la ligne d'inspection peut être saisie de préférence simultanément par la première caméra (11, 11', 11", 14') et par la deuxième caméra (12, 12', 15'), sachant que la première caméra (11, 11', 11", 14') et la deuxième caméra (12, 12', 15') sont agencées de telle manière qu'elles déterminent respectivement la valeur chromatique de la lumière réfléchie par chaque point de la ligne d'inspection, sachant que le système d'évaluation relié à la première caméra et à la deuxième caméra est agencé de telle manière qu'il utilise les valeurs chromatiques de la première caméra et de la deuxième caméra à chaque point de la ligne d'inspection pour une comparaison avec la valeur chromatique théorique déterminée, prédéfinie ou la plage de valeurs chromatiques théoriques déterminée, prédéfinie, sachant que le système d'évaluation est en plus agencé de telle manière qu'il évalue en plus la valeur chromatique de la lumière réfléchie de différents points de la ligne d'inspection en fonction des angles d'observation correspondants de ceux-ci.

2. Dispositif selon la revendication 1, **caractérisé en ce que** le système d'évaluation est agencé de telle manière que les différences de la première valeur chromatique du premier point de la ligne d'inspection et de la deuxième valeur chromatique du deuxième point de la ligne d'inspection sont comparées à la valeur théorique déterminée, prédéfinie ou à la plage de valeurs chromatiques théoriques déterminée, prédéfinie, sachant que le premier point et le deuxième point sont observés par la caméra dans différentes directions le long de la ligne d'inspection sous le même ou à peu près le même angle d'observation.

3. Dispositif selon l'une quelconque des revendications précédentes, **caractérisé en ce que** la première caméra (11, 11', 11", 14') et au moins une deuxième caméra (12, 12', 15') sont disposées sur un support commun (5) .

4. Dispositif selon l'une quelconque des revendications précédentes, **caractérisé en ce que** la première caméra (11, 11', 11", 14') et/ou la deuxième caméra (12, 12', 15') est respectivement une caméra linéaire ou une caméra matricielle.

5. Dispositif selon l'une quelconque des revendications précédentes, **caractérisé en ce que** le premier angle d'observation et le deuxième angle d'observation divergent d'au moins 10°, de préférence d'au moins 15°, de préférence en particulier d'au moins 20°.

6. Dispositif selon l'une quelconque des revendications précédentes, **caractérisé en ce que** le matériau (2) peut être déplacé par rapport à la première caméra (11, 11', 11", 14') et à la deuxième caméra (12, 12', 15') et par rapport à la source lumineuse (18) en direction d'un sens d'avance (3), sachant de préférence que la source lumineuse (18) et/ou la première caméra (11, 11', 11", 14') et/ou la deuxième caméra (12, 12', 15') sont inclinées dans le sens d'avance ou contrairement au sens d'avance.

7. Dispositif selon l'une quelconque des revendications précédentes, **caractérisé en ce qu'** au moins une troisième caméra (13, 13', 16') est prévue, laquelle est agencée pour la détermination d'une valeur chromatique de la lumière saisie, sachant que la troisième caméra (13, 13', 16') est disposée au-dessus de la ligne d'inspection et inclinée vers la source lumineuse (18), sachant que la troisième caméra possède un troisième angle d'ouverture de telle manière que la lumière réfléchie de la source lumineuse (18) peut être en plus saisie au moins pour les points de la ligne d'inspection dans un secteur déterminé de la ligne d'inspection et la valeur chromatique correspondante peut être déterminée,
sachant que le système d'évaluation compare pour chaque point du secteur déterminé de la ligne d'inspection, l'angle d'observation de la troisième caméra à l'angle d'observation de la première caméra (11, 11', 11", 14') et/ou à l'angle d'observation de la deuxième caméra (12, 12', 15') et utilise pour le point correspondant du secteur déterminé les deux valeurs chromatiques de la première caméra (11, 11', 11", 14'), de la deuxième caméra (12, 12', 15') et de la troisième caméra (13, 13', 16') pour une comparaison avec la valeur chromatique théorique déterminée, prédéfinie ou la plage de valeurs chromatiques théoriques déterminée, prédéfinie pour lesquelles la différence des angles d'observation correspondants est la plus grande.

8. Dispositif selon l'une quelconque des revendications précédentes, **caractérisé en ce que** le système d'évaluation détermine à chaque point de la ligne d'inspection respectivement une différence de valeur chromatique de deux valeurs chromatiques de deux caméras déterminées, qui observent la lumière réfléchie de ce point et compare cette différence de valeur chromatique à une valeur théorique de différence de valeur chromatique déterminée, prédéfinie ou à une plage de valeurs théoriques de différence de valeur chromatique déterminée, prédéfinie.

9. Procédé d'inspection d'un matériau (2) doté d'une surface revêtue, de préférence du verre, sachant que sont prévues une source lumineuse (18) disposée au-dessus de la surface, laquelle délivre de la lumière dans une plage de longueurs d'ondes prédéfinie en direction de la surface,
et une première caméra (11, 11', 11", 14'), sachant que la première caméra (11, 11', 11", 14') est agencée pour la détermination d'une valeur chromatique de la lumière saisie, sachant que la première caméra (11, 11', 11", 14') est disposée au-dessus d'une ligne d'inspection située sur la surface, inclinée vers la source lumineuse de telle manière qu'elle voit la réflexion réfléchissante de la lumière émise par la source lumineuse sur la surface revêtue dans le secteur de la ligne d'inspection et possède un premier angle d'ouverture (21), avec les étapes suivantes :
• au moyen de la première caméra (11, 11', 11", 14'), la lumière de la source lumineuse (18), réfléchie par un premier point de la ligne d'inspection est séparément saisie sous un premier angle d'observation et celle par un deuxième point distant du premier point de la ligne d'inspection sous un deuxième angle d'observation, sachant que le premier angle d'observation et le deuxième angle d'observation sont différents, sachant que la première caméra détermine une première valeur chromatique de la lumière réfléchie par le premier point et une deuxième valeur chromatique de la lumière réfléchie par le deuxième point,
• sachant qu'au moyen d'un système d'évaluation relié à la première caméra (11, 11', 11", 14'), la première valeur chromatique et la deuxième valeur chromatique sont respectivement comparées à une valeur chromatique théorique déterminée, prédéfinie ou à une plage de valeurs chromatiques théoriques déterminée, prédéfinie ou une différence de la première valeur chromatique et de la deuxième valeur chromatique est comparée à une valeur théorique de différence de valeur chromatique déterminée, prédéfinie ou à une plage de valeurs théoriques de différence de valeur chromatique déterminée, prédéfinie,
sachant qu'au moins une deuxième caméra (12, 12', 15') est prévue, sachant que la deuxième caméra (12, 12', 15') est également agencée pour la détermination d'une valeur chromatique de la lumière saisie,
sachant que la deuxième caméra (12, 12', 15') est disposée au-dessus de la ligne d'inspection située sur la surface et inclinée vers la source lumineuse, sachant que la deuxième caméra (12, 12', 15') possède un deuxième angle d'ouverture (22), sachant que la lumière de la source lumineuse (18), réfléchie par chaque point de la ligne d'inspection est de préférence simultanément saisie par la première caméra (11, 11', 11", 14') et par la deuxième caméra (12, 12', 15') et la valeur chromatique de la lumière réfléchie par chaque point de la ligne d'inspection est respectivement déterminée et est comparée par le système d'évaluation à la valeur chromatique théorique déterminée, prédéfinie ou à la plage de valeurs chromatiques théoriques déterminée, prédéfinie, sachant qu'au moyen du système d'évaluation, la valeur chromatique de la lumière réfléchie de différents points de la ligne d'inspection est évaluée en plus en fonction des angles d'observation correspondants de ceux-ci.

10. Procédé selon la revendication 9, **caractérisé en ce qu'**au moins une troisième caméra (13, 13', 16') est prévue, laquelle est agencée pour la détermination d'une valeur chromatique de la lumière saisie, sachant que la troisième caméra (13, 13', 16') est disposée au-dessus de la ligne d'inspection et inclinée vers la source lumineuse (18), sachant que la troisième caméra (13, 13', 16') possède un troisième angle d'ouverture (23), sachant que la lumière réfléchie de la source lumineuse (18) est en plus saisie par la troisième caméra (13, 13', 16') au moins pour les points de la ligne d'inspection dans un secteur déterminé de la ligne d'inspection et la valeur chromatique correspondante est déterminée, sachant que l'angle d'observation de la troisième caméra (13, 13', 16') est comparé par le système d'évaluation pour chaque point du secteur déterminé de la ligne d'inspection à l'angle d'observation de la première caméra (11, 11', 11", 14') et/ou à l'angle d'observation de la deuxième caméra (12, 12', 15') et pour le point correspondant du secteur déterminé, les deux valeurs chromatiques de la première caméra (11, 11', 11", 14'), de la deuxième caméra (12, 12', 15') et de la troisième caméra (13, 13', 16') sont utilisées pour la comparaison à la valeur chromatique théorique déterminée, prédéfinie ou la plage de valeurs chromatiques théoriques déterminée, prédéfinie pour lesquelles la différence des angles d'observation correspondants est la plus grande.

11. Procédé selon l'une quelconque des revendications 9 à 10, **caractérisé en ce qu'**au moyen du système d'évaluation, une différence de valeur chromatique est respectivement déterminée dans chaque point de la ligne d'inspection par deux valeurs chromatiques de deux caméras déterminées, qui observent la lumière réfléchie de ce point et cette différence de valeur chromatique est comparée à une valeur théorique de différence de valeur chromatique déterminée, prédéfinie ou à une plage de valeurs théoriques de différence de valeur chromatique déterminée, prédéfinie.
